# EUROPEAN PATENT APPLICATION

(11) **EP 4 272 736 A1**
(43) Date of publication of application: **08.11.2023**
(21) Application number: 21914456.5
(22) Date of filing: 29.12.2021
(51) Int. Cl.: A61K 31/222, A61K 9/72, A61K 9/10, A61P 11/00

(54) **PHARMACEUTICAL COMPOSITION FOR INHALATION**

(30) Priority: 31.12.2020 CN 202011622511
(71) Applicant: Shanghai Huilun Pharmaceutical Co., Ltd., Shanghai 201108 (CN); Shanghai Huilun Jiangsu Pharmaceutical Co., Ltd., Taizhou, Jiangsu 225300 (CN)
(72) Inventor: LI, Changjun, Shanghai 201108 (CN); LI, Wenhua, Shanghai 201108 (CN); QIN, Jihong, Shanghai 201108 (CN); LIU, Huali, Shanghai 201108 (CN); LI, Jinfeng, Shanghai 201108 (CN); NIE, Xiangjiang, Shanghai 201108 (CN)
(74) Representative: Regimbeau
(86) International application number: PCT/CN2021/142306
(87) International publication number: WO 2022/143722

(57) **Abstract**

The present invention relates to a pharmaceutical composition for inhalation, comprising N-[2-[[[4-(2,2-dimethyl-1-oxopropoxy)phenyl]sulfonyl]amino]benzoyl]-(S)-glycinate sodium or a hydrate thereof, a pH adjuster, an osmotic pressure adjuster, and optionally a surfactant. The pharmaceutical composition for inhalation of the present invention can improve drug stability. After being atomized by an inhalation atomizing device, inhalation droplets can maintain good uniformity.

## Description

### TECHNICAL FIELD

The present application relates to the field of medicine, and in particular to a pharmaceutical composition of sivelestat sodium or a hydrate thereof for inhalation.

### BACKGROUND

Sivelestat (N-[2-[[[4-(2,2-dimethyl-1-oxopropoxy)phenyl]sulfonyl]amino]benzoyl]-(S)-glycine) is an inhibitor of neutrophil elastase, which is used to treat acute lung injury or acute respiratory distress syndrome of systemic inflammatory response syndrome. It is usually used in the form of sodium salt tetrahydrate. Sivilestat has the following structure:

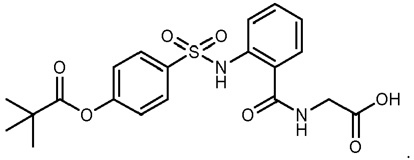

The known commercial sivelestat sodium is an injection preparation, and the formula of the preparation contains mannitol, sodium hydroxide and other auxiliary materials. At present, sivelestat sodium is only available for injection. Patients need to be continuously medicated for a long time, such as 24-hour continuous drip, and patients with acute lung injury are usually seriously ill, which makes the administration difficult. Therefore, it is urgent to develop a simple and convenient administration method.

Document 1 discloses a dry powder inhalant of sivelestat sodium, which can be inhaled through oral or nasal cavity. The inhalant is added with an antistatic agent, such as L-glucoside, cyclodextrin, sorbitol, chitin, sodium hydroxymethyl starch, etc., and a surface active ingredient, such as poloxamer, dilauroyl phosphatidylcholine, etc., and other auxiliary materials. It is also disclosed that the inhalant can quickly provide pharmaceutical effects and effectively increase medical uses. However, if the lyophilized powder of sivelestat sodium injection is directly inhaled, the mannitol contained can easily cause side effects such as airway narrowing and asthma; thus, the lyophilized powder cannot be directly used for inhalation, which is especially not suitable for direct inhalation by human body.

Document 2 discloses a sustained-release microsphere for treating lung diseases, and the sustained-release microsphere has an average particle size of 20-40 µm. Drugs that can be used in the sustained-release microsphere include sivelestat sodium.

Document 3 describes a lyophilized preparation of sivelestat sodium for injection. The inactive ingredients contain a pH-adjusting agent composed of sodium dihydrogen phosphate and sodium hydroxide, and excipient lactose or mannitol. Similarly, the lyophilized powder cannot be directly used for inhalation, which is especially not suitable for direct inhalation by human body.

Document 4 discloses a lyophilized preparation of sivelestat sodium for injection, including a pH-adjusting agent selected from trisodium phosphate and its hydrate, sodium hydroxide or potassium hydroxide.

Document 5 discloses a lyophilized preparation of sivelestat sodium for injection containing anhydrous sodium carbonate as a pH-adjusting agent and sodium chloride. According to the description in Document 4, it has been proved that the pH of the lyophilized preparation rises during this process, and a large number of decomposed products are obtained. When the lyophilized product is kept at 60°C for two weeks, the residual rate of sivelestat sodium will only be 91.4%.

The preparation of sivelestat sodium reported in the existing documents is either lyophilized powder for injection or dry powder inhalant. The existing art cannot meet the clinical needs. For patients, there is an urgent need for a preparation of sivelestat sodium, which can avoid continuous injection and is convenient to use, and can achieve the expected therapeutic effect.
Document 1: CN107913261A
Document 2: EP291304
Document 3: CN104107172A
Document 4: CN1263736C
Document 5: US7638556B2

### SUMMARY

The solubility of sivelestat sodium in water is less than 0.4 mg/mL, which cannot meet the administration requirements as an injection or inhalation preparation. Besides, sivelestat sodium is prone to hydrolysis in aqueous solution, generating the impurity with the following structure (hereinafter referred to as "impurity A").

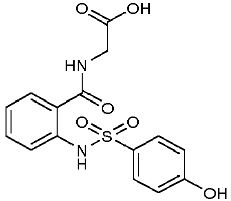

In the study, it is found that sivelestat sodium has a better solubility in the aqueous solution with higher pH; however, with the increase of pH, the hydrolysis degree of sivelestat sodium also increases obviously. The relationship among solubility, pH and hydrolysis degree of sivelestat sodium in lyophilized preparation for injection has been described in the prior art. However, the prior art has no description about the pharmaceutical composition of sivelestat sodium for inhalation, especially about the pharmaceutical composition that can be prepared into an inhalation solution.

The pharmaceutical composition for inhalation does not contain mannitol, an auxiliary material of lyophilized preparation for injection, and the weight ratio of sivelestat sodium or a hydrate thereofin the pharmaceutical composition for inhalation is increased, which will have a significant impact on the stability of the pharmaceutical composition for inhalation, such as the increase of impurity content, and the droplets after atomization will have poor uniformity, and the droplet size will be difficult to control.

In order to solve the above problems, the present application provides a pharmaceutical composition for inhalation, which includes sivelestat sodium or a hydrate thereof, a pH-adjusting agent and an osmotic pressure-adjusting agent.

The pH-adjusting agent provided by the present application is capable to keep sivelestat sodium contained in the pharmaceutical composition having a solubility of more than or equal to 2.5 mg/mL; and/or the pH-adjusting agent is capable to keep sivelestat sodium contained in the pharmaceutical composition having a solubility of more than or equal to 5 mg/mL; and/or the pH-adjusting agent is capable to keep sivelestat sodium contained in the pharmaceutical composition having a solubility of more than or equal to 7.5 mg/mL; and/or the pH-adjusting agent is capable to keep sivelestat sodium contained in the pharmaceutical composition having a solubility of more than or equal to 10.0 mg/mL; and/or the pH-adjusting agent is capable to keep sivelestat sodium contained in the pharmaceutical composition having a solubility of more than or equal to 15.0 mg/mL; and/or the pH-adjusting agent is capable to keep sivelestat sodium contained in the pharmaceutical composition having a solubility of more than or equal to 20.0 mg/mL.

The pH-adjusting agent of the present application is a pharmaceutically acceptable acid, alkali and corresponding salt or a buffer system including any combination of acid, alkali and salt. The pH-adjusting agent unrestrictedly includes an organic base, an organic acid and a corresponding salt, and an inorganic acid, an inorganic base and a corresponding salt. The inorganic acid and the salt include hydrochloric acid, phosphoric acid, phosphate, carbonate and bicarbonate. The organic base includes, for example, diethanolamine, triethanolamine and tromethamine; the organic acid includes, for example, acetic acid, lactic acid, citric acid, fumaric acid, tartaric acid; and corresponding salts; the commonly used carbonate includes, for example, sodium carbonate, calcium carbonate and ammonium carbonate; the commonly used bicarbonate includes, for example, sodium bicarbonate and potassium bicarbonate; the inorganic base includes, for example, alkali metal hydroxide and alkaline earth metal hydroxide; the phosphate includes, for example, sodium phosphate, sodium hydrogen phosphate, sodium dihydrogen phosphate and a hydrate thereof; the commonly used alkali metal hydroxide includes, for example, sodium hydroxide, and potassium hydroxide.

The pH-adjusting agent of the present application is capable to keep the pharmaceutical composition for inhalation at pH 3.0-10.0; as a preferred solution, the pH ranges from 4.0 to 9.5; or the pH ranges from 5.0 to 9.0; or the pH ranges from 5.5 to 8.5. The pH-adjusting agent is used in the present application to give the pharmaceutical composition for inhalation containing sivelestat sodium excellent solubility.

The pH-adjusting agent of the present application has a mass-volume ratio (w/v, mg/mL) of 0.1-30.0 mg/mL in the solution; as a preferred solution, the pH-adjusting agent of the present application has a mass-volume ratio of 0.1-25.0 mg/mL; as a more preferred solution, the pH-adjusting agent of the present application has a mass-volume ratio of 0.5-20.0 mg/mL.

The osmotic pressure-adjusting agent for the pharmaceutical composition for inhalation of the present application unrestrictedly includes sodium chloride, potassium chloride, glucose, calcium chloride, magnesium chloride, sorbitol, and xylitol; as a preferred solution of the present application, the osmotic pressure-adjusting agent is selected from sodium chloride and glucose. The osmotic pressure-adjusting agent of the present application is capable to keep an osmolality of the pharmaceutical composition for inhalation at 250-450 mOsm/kg; or the osmotic pressure-adjusting agent is capable to keep an osmolality of the pharmaceutical composition for inhalation at 250-400 mOsm/kg; or the osmotic pressure-adjusting agent is capable to keep an osmolality of the pharmaceutical composition for inhalation at 250-350 mOsm/kg. Within the osmotic pressure range, the pharmaceutical composition for inhalation of the present application shows good stability.

The pharmaceutical composition for inhalation of the present application optionally includes a surfactant. The surfactant has a content of 0.01-10.0 mg/mL; as a preferred solution, the surfactant has a content of 0.01-5.0 mg/mL; as a more preferred solution, the surfactant has a content of 0.01-3.5 mg/mL.

The surfactant of the present application is a nonionic surfactant or an ionic surfactant. As an optional surfactant, non-limiting examples include: ethylene glycol monostearate, ethylene glycol monooleate, sodium dodecylbenzene sulfonate, sodium dodecyl sulfate, glycerol trioleate, sorbitan monolaurate, poloxamer, polysorbate-20, polysorbate-40, polysorbate-60, polysorbate-80, and polyoxyethylene castor oil. As a preferred solution of the present application, the surfactant is optionally selected from sodium dodecyl sulfate and polysorbate-80.

Another aspect of the present application provides a pharmaceutical composition for inhalation, which includes sivelestat sodium or a hydrate thereof, a pH-adjusting agent and an osmotic pressure-adjusting agent, wherein the pH-adjusting agent is capable to keep sivelestat sodium contained in the pharmaceutical composition having a solubility of more than or equal to 2.5 mg/mL, and the pharmaceutical composition for inhalation has an osmolality of 250-400 mOsm/kg, and the pharmaceutical composition for inhalation of the present application has at least one of the following features:
(1) the pharmaceutical composition for inhalation has a pH of 3.0-10.0;
(2) the pharmaceutical composition for inhalation has a total impurity content of less than or equal to 2.0%;
(3) a surfactant is optionally included.

Another aspect of the present application provides a pharmaceutical composition for inhalation, which includes sivelestat sodium or a hydrate thereof, a pH-adjusting agent, an osmotic pressure-adjusting agent and a surfactant, wherein the pH-adjusting agent is capable to keep sivelestat sodium contained in the pharmaceutical composition having a solubility of at more than or equal to 2.5 mg/mL, and the pharmaceutical composition for inhalation has an osmolality of 250-400 mOsm/kg, and the surfactant has a content of 0.01-10.0 mg/mL.

The pharmaceutical composition for inhalation of the present application can be administered once, twice, three times or more per day. As a preferred solution, each dose should be less than or equal to 500 mg, and a total daily dose should be less than or equal to 2000 mg/day.

The pharmaceutical composition for inhalation of the present application is administered by inhalation after being dissolved in a carrier and atomized by an existing conventional atomizing device. Therefore, the present application also provides a medicine box, which contains the pharmaceutical composition for inhalation including sivelestat sodium or a hydrate thereof and a certain dose of water, wherein the dose is 1 mL, 2 mL, 3 mL, 4 mL, 5 mL or more. Before use, the pharmaceutical composition for inhalation of the present application is mixed and dissolved with water, such as distilled water and water for injection, then atomized by an administration device and then administered by inhalation, wherein the administration device can be any atomizer capable of delivering drug to the nidus.

Another aspect of the present application provides a pharmaceutical composition for inhalation which can be redissolved within a predetermined time, wherein the redissolving time is less than 60 s, or less than 50 s, or less than 40 s, or less than 35 s, or less than 30 s, or less than 25 s, or less than 20 s. The composition includes sivelestat sodium or a hydrate thereof, a pH-adjusting agent, an osmotic pressure-adjusting agent and optionally a surfactant. The pH-adjusting agent is capable to keep sivelestat sodium contained in the pharmaceutical composition having a solubility of more than or equal to 2.5 mg/mL, and an osmolality is 250-400 mOsm/kg, and the surfactant has a content of 0.01-10.0 mg/mL. As a preferred solution of the present application, the pharmaceutical composition is redissolved in less than 40 s; as a preferred solution of the present application, the pharmaceutical composition is redissolved in less than 30 s.

Another aspect of the present application provides a pharmaceutical composition for inhalation for treating lung diseases, which includes sivelestat sodium or a hydrate thereof, a pH-adjusting agent, an osmotic pressure-adjusting agent and optionally a surfactant. The pharmaceutical composition is mixed with a carrier, such as water, normal saline, etc., atomized by an inhaler and inhaled. The inhaled atomized particles (or droplets) have a mass median aerodynamic diameter (MMAD) of 0.1-15.0 µm; as a preferred solution of the present application, the inhaled atomized particles have a mass median aerodynamic diameter (or particle size) of 0.1-10.0 µm; as a further preferred solution of the present application, the inhaled atomized particles have a mass median aerodynamic diameter of 0.5-7.0 µm; as a further preferred solution of the present application, the inhaled atomized particles have a mass median aerodynamic diameter of 0.5-5.0 µm. As a further preferred solution of the present application, the inhaled atomized particles with a mass median aerodynamic diameter of less than 5.0 µm has a proportion (or fine particle fraction) of more than or equal to 50%; as a further preferred solution, the inhaled atomized particles with a mass median aerodynamic diameter of less than 5.0 µm has a proportion (or fine particle fraction) of more than or equal to 60%; as a further preferred solution, the inhaled atomized particles with a mass median aerodynamic diameter of less than 5.0 µm has a proportion (or fine particle fraction) of more than or equal to 70%.

Another aspect of the present application provides a pharmaceutical composition for inhalation, which includes sivelestat sodium or a hydrate thereof, a pH-adjusting agent, an osmotic pressure-adjusting agent and a surfactant, wherein the pH-adjusting agent is capable to keep sivelestat sodium or the hydrate thereof contained in the pharmaceutical composition having a solubility of more than or equal to 5 mg/mL, and the pharmaceutical composition for inhalation has an osmolality of 250-400 mOsm/kg, and the pharmaceutical composition for inhalation of the present application has at least one of the following features:
(1) the pharmaceutical composition for inhalation has a pH of 5.0-9.0;
(2) the pharmaceutical composition for inhalation has a total impurity content of less than or equal to 1.5%;
(3) the pharmaceutical composition for inhalation has a particle size of 0.5-7.0 µm after being atomized by an atomizing device;
(4) the pharmaceutical composition for inhalation has a redissolving time of less than or equal to 30 s in a solution.

Another aspect of the present application provides a therapeutic method for a disease and use of a pharmaceutical composition for inhalation in preparing drugs for treating a disease, wherein the disease is caused by elastase, and the disease is a lung disease; the lung disease includes acute lung injury (ALL) or acute respiratory distress syndrome (ARDS). The therapeutic method or use is to administer a certain dose of the pharmaceutical composition of sivelestat sodium or a hydrate thereof for inhalation, wherein a dose is less than or equal to 500 mg each time, and a total daily dose is less than or equal to 2000 mg/day.

Another aspect of the present application provides a method of preparing a pharmaceutical composition for inhalation, which includes dissolving a pH-adjusting agent, an osmotic pressure-adjusting agent, sivelestat sodium and/or a surfactant in water, vialing and then lyophilizing.

It is found through experiments in the present application that the surfactant is not conducive to the stability of sivelestat sodium, and the addition of the surfactant will increase the impurity content of sivelestat sodium or a hydrate thereofin the pharmaceutical composition. By controlling the content of each component in the pharmaceutical composition in the present application, the pharmaceutical composition with good stability can be obtained, and for example, a total impurity content is less than or equal to 2.0%, or less than or equal to 1.5%; the pharmaceutical composition for inhalation can be redissolved within a predetermined time (for example, less than 30 s), and maintain good uniformity in the particle size range after atomization.

For patients with acute lung injury or acute respiratory distress syndrome, the particle size of inhaled sivelestat sodium or a hydrate thereof after atomization needs to be within a specific range (0.5-7.0 µm) to facilitate drug depositing in the alveoli. If the particle size is too small, the atomized particles can easily be exhaled and lose the therapeutic effect; if the particle size is too large, the atomized particles are mainly deposited in the upper respiratory tract, which affects the absorption site of drugs. Improper particle size will directly affect the absorption and therapeutic effects of the drug and increase potential side effects. Therefore, the pharmaceutical composition for inhalation of the present application not only has good stability, but also has a uniform particle size distribution within 0.5-7.0 µm after atomization by an atomizer, which can obtain a good therapeutic effect.

### Terminology explanation

The "hydrate" in the present application refers to monohydrate, dihydrate, trihydrate, tetrahydrate, etc. Preferably, sivelestat sodium exists in the form of tetrahydrate.

### DETAILED DESCRIPTION

The embodiments of the present application are used to illustrate the technical solutions of the present application unrestrictedly, and should not be construed as limitations of the present application. The drugs used in the present application can be prepared by the methods reported in the existing documents, and the reagents used are all commercially available unless otherwise specified. In the present application, the impurity content of the composition is determined by a high performance liquid chromatograph, the pH of the solution is detected by a pH measuring instrument, and the osmotic pressure of the solution is measured by an osmotic pressure measuring instrument. The term "MMAD" in the present application refers to the mass median aerodynamic diameter.

### Reference Example 1

With reference to a method in patent CN104107172, a mannitol aqueous solution of 40 mg/ml was prepared, and sivelestat sodium tetrahydrate was added as 20 mg/ml and dispersed; the bulk drug was dissolved with a sodium dihydrogen phosphate-sodium hydroxide buffer, and the drug solution had a pH of 7.6, and was vialed as 5 mL and lyophilized to obtain a finished product.

The lyophilized finished product was placed at a high temperature of 60°C to evaluate the stability. When the sample was redissolved at day 0, a pH was 7.6, a main degradation impurity A content was 0.7% and a total impurity content was 0.8%; when the sample was redissolved after 10 days at a high temperature of 60°C, a pH was 7.6, a impurity A content was 1.6% and a total impurity content was 1.8%.

### Reference Example 2

With reference to a method disclosed in patent CN1263736C, a mannitol aqueous solution of 40 mg/ml was prepared, and sivelestat sodium tetrahydrate was added as 20 mg/ml and dispersed; the bulk drug was dissolved with a trisodium phosphate-sodium hydroxide buffer, and the drug solution had a pH of 7.8, and was vialed as 5 mL and lyophilized.

The lyophilized finished product was placed at a high temperature of 60°C to evaluate the stability. When the sample was redissolved at day 0, a pH was 7.8, a main degradation impurity A content was 1.0% and a total impurity content was 1.3%; when the sample was redissolved after 10 days at a high temperature of 60°C, a pH was 7.9, a impurity A content was 1.5% and a total impurity content was 1.7%.

### Example 1

Formula preparation process: sodium chloride and sodium citrate were added into an aqueous solution in sequence as concentrations of 6 mg/ml and 6 mg/ml, respectively, sodium hydroxide was 0.17 mg/ml, and sivelestat sodium tetrahydrate was added as 5 mg/ml; the system was stirred to dissolve and clarify, and the drug solution had a pH of 7.2, and was vialed as 5 mL and lyophilized.

The lyophilized finished product was placed at a high temperature of 60°C to evaluate the stability. When the sample was redissolved at day 0, a pH was 7.2, a main degradation impurity A content was 0.20% and a total impurity content was 0.37%; when the sample was redissolved after 10 days at a high temperature of 60°C, a pH was 7.3, a impurity A content was 0.31% and a total impurity content was 0.52%.

### Example 2

The drug solution was prepared according to the same method as in Example 1, and added with polysorbate-80 as 0.2 mg/ml, vialed as 5 mL and lyophilized.

The lyophilized finished product was placed at a high temperature of 60°C to evaluate the stability. When the sample was redissolved at day 0, a pH was 7.3, a main degradation impurity A content was 0.22% and a total impurity content was 0.38%; when the sample was redissolved after 10 days at a high temperature of 60°C, a pH was 7.2, a impurity A content was 0.72% and a total impurity content was 0.98%.

### Example 3

Preparation process of formula for inhalation: sodium chloride and sodium citrate were added into an aqueous solution in sequence as concentrations of 6 mg/ml and 6 mg/ml, respectively, sodium hydroxide was 0.34 mg/ml, and sivelestat sodium tetrahydrate was added as 7.5 mg/ml; the system was stirred to dissolve and clarify, and the drug solution had a pH of 7.4, and was vialed as 5 mL and lyophilized.

### Example 4

The drug solution of Example 3 was added with polysorbate-80 as 0.2 mg/ml, and vialed and lyophilized.

The stability results of Examples 1-4 and Reference Example 1 are shown in the table below.

| Reference Example 1 | | | Example | | | | | |
|---|---|---|---|---|---|---|---|---|
| Formula | | Reference Example | Formula | | Example 1 | Example 2 | Example 3 | Example 4 |
| sivelestat sodium | | 20 mg | sivelestat sodium | | 5 mg | 5 mg | 7.5 mg | 7.5 mg |
| mannitol | | 40 mg | sodium chloride | | 6 mg | 6 mg | 6 mg | 6 mg |
| sodium bicarbonate | | appropriate amount | sodium citrate | | 6 mg | 6 mg | 6 mg | 6 mg |
| sodium hydroxide | | appropriate amount | sodium hydroxide | | 0.17 mg | 0.17 mg | 0.34 mg | 0.34 mg |
| - | | - | polysorbate-80 | | - | 0.2 mg | - | 0.2 mg |
| water | | 1 ml | water | | 1 ml | 1 ml | 1 ml | 1 ml |
| pH of drug solution | | 7.6 | pH of drug solution | | 7.2 | 7.2 | 7.4 | 7.4 |

| Quality evaluation of lyophilized sample | | | | | | | | |
|---|---|---|---|---|---|---|---|---|
| Evaluated item | | Reference Example | Evaluated item | | Example 1 | Example 2 | Example 3 | Example 4 |
| Redissolving time | | 35 s | Redissolving time | | 28 s | 18 s | 26 s | 20 s |
| Day 0 | osmotic pressure | 352 | Day 0 | osmotic pressure | 267 | 250 | 265 | 260 |
| | pH | 7.6 | | pH | 7.2 | 7.3 | 7.4 | 7.4 |
| | impurity A | 0.7% | | impurity A | 0.20 | 0.22 | 0.16 | 0.17 |
| | total impurities | 0.8% | | total impurities | 0.37 | 0.38 | 0.33 | 0.34 |
| 60°C, 10 d | pH | 7.6 | 60°C, 10 d | pH | 7.3 | 7.2 | 7.4 | 7.4 |
| | Impurity A | 1.6% | | impurity A | 0.31 | 0.72 | 0.25 | 0.51 |
| | Total impurities | 1.8% | | total impurities | 0.52 | 0.98 | 0.42 | 0.73 |

Note: the osmotic pressure is in mOsmol/kg, and all the lyophilized samples are redissolved with water to a dosed the concentration. The redissolving time refers to the time required to redissolve the lyophilized preparation in 5 mL aqueous solution.

The pharmaceutical composition for inhalation of the present application can significantly improve the stability of the drug, reducing the impurity A content and the total impurity content, and the pH is stable at day 0 and day 10. For the formula containing polysorbate-80, the redissolving time can be further shortened.

### Example 5

Preparation process of formula for inhalation: a 6 mg/ml sodium chloride aqueous solution was prepared, tromethamine was 0.5 mg/ml, sivelestat sodium tetrahydrate was added as 5 mg/ml, and the system was stirred to dissolve and clarify; the drug solution had a pH of 7.2, and was vialed as 5 mL and lyophilized.

The lyophilized finished product was placed at a high temperature of 60°C to evaluate the stability. When the sample was redissolved at day 0, a pH was 7.2, a main degradation impurity A content was 0.13% and a total impurity content was 0.25%; when the sample was redissolved after 10 days at a high temperature of 60°C, a pH was 7.2, a impurity A content was 0.28% and a total impurity content was 0.49%.

According to the same preparation process, when tromethamine was 0.242 mg/ml, the drug could not be completely dissolved and clarified.

### Example 6

Preparation process of formula for inhalation: a 6 mg/ml sodium chloride aqueous solution was prepared, sodium carbonate was 10 mg/ml, sivelestat sodium tetrahydrate was added as 20 mg/ml, and the system was stirred to dissolve and clarify; the drug solution had a pH of 9.0, and was vialed as 5 mL and lyophilized.

The lyophilized finished product was placed at a high temperature of 60°C to evaluate the stability. When the sample was redissolved at day 0, a pH was 9.1, a main degradation impurity A content was 0.44% and a total impurity content was 0.57%; when the sample was redissolved after 10 days at a high temperature of 60°C, a pH was 9.5, a impurity A content was 1.03% and a total impurity content was 1.12%.

### Example 7

Preparation process of formula for inhalation: a 10 mg/ml sodium bicarbonate aqueous solution was prepared, sivelestat sodium tetrahydrate was added as 20 mg/ml, and the system was stirred to dissolve and clarify; the drug solution had a pH of 8.1, and was vialed and lyophilized.

### Example 8

Preparation process of formula for inhalation: the drug solution of Example 7 was added with sodium chloride as 4 mg/ml and polysorbate-80 as 0.2 mg/ml, and stirred to dissolve and clarify; the drug solution had a pH of 8.1, and was vialed and lyophilized.

### Example 9

Preparation process of formula for inhalation: a 6 mg/ml sodium bicarbonate aqueous solution was prepared, sivelestat sodium tetrahydrate was added as 20 mg/ml, and the system was stirred to dissolve and clarify; the drug solution had a pH of 7.8, and was vialed and lyophilized.

### Example 10

Preparation process of formula for inhalation: the drug solution of Example 9 was added with sodium chloride as 4 mg/ml and polysorbate-80 as 0.2 mg/ml, and stirred to dissolve and clarify; the drug solution had a pH of 7.8, and was vialed and lyophilized.

### Example 11

Preparation process of formula for inhalation: the drug solution of Example 9 was added with sodium chloride as 8 mg/ml and polysorbate-80 as 0.2 mg/ml, and stirred to dissolve and clarify; the drug solution had a pH of 7.8, and was vialed and lyophilized.

### Example 12

Preparation process of formula for inhalation: a 4 mg/ml sodium bicarbonate aqueous solution was prepared, sivelestat sodium tetrahydrate was added as 10 mg/ml, and the system was stirred to dissolve and clarify; the drug solution had a pH of 7.8, and was vialed as 5 mL and lyophilized.

### Example 13

Preparation process of formula for inhalation: the drug solution of Example 12 was added with sodium chloride as 4 mg/ml and polysorbate-80 as 0.2 mg/ml, and stirred to dissolve and clarify; the drug solution had a pH of 7.8, and was vialed as 5 mL and lyophilized.

### Example 14

Preparation process of formula for inhalation: the drug solution of Example 12 was added with sodium chloride as 8 mg/ml and polysorbate-80 as 0.2 mg/ml, and stirred to dissolve and clarify; the drug solution had a pH of 7.8, and was vialed as 5 mL and lyophilized.

The stability results of Examples 7-14 are shown in the table below.

| Formula | | Example 7 | Example 8 | Example 9 | Example 10 | Example 11 | Example 12 | Example 13 | Example 14 |
|---|---|---|---|---|---|---|---|---|---|
| sivelestat sodium | | 20 mg | 20 mg | 20 mg | 20 mg | 20 mg | 10 mg | 10 mg | 10 mg |
| sodium bicarbonate | | 10 mg | 10 mg | 6 mg | 6 mg | 6 mg | 4 mg | 4 mg | 4 mg |
| sodium chloride | | - | 4 mg | - | 4 mg | 8 mg | - | 4 mg | 8 mg |
| polysorbate-80 | | - | 0.2 mg | - | 0.2 mg | 0.2 mg | - | 0.2 mg | 0.2 mg |
| water | | 1 ml | 1 ml | 1 ml | 1 ml | 1 ml | 1 ml | 1 ml | 1 ml |
| pH of drug solution | | 8.1 | 8.1 | 7.8 | 7.8 | 7.8 | 7.8 | 7.8 | 7.8 |

| Quality evaluation of lyophilized sample | | | | | | | | | |
|---|---|---|---|---|---|---|---|---|---|
| Evaluated item | | Example 7 | Example 8 | Example 9 | Example 10 | Example 11 | Example 12 | Example 13 | Example 14 |
| Redissolving time | | 30 s | 20 s | 28 s | 17s | 18 s | 25 s | 20 s | 19s |
| Day 0 | osmot ic pressu re | 104 | 285 | 121 | 274 | 304 | 109 | 265 | 297 |
| | pH | 8.1 | 8.1 | 8.0 | 7.9 | 7.9 | 7.9 | 8.0 | 8.0 |
| | impuri tyA | 0.30 | 0.28 | 0.21 | 0.18 | 0.17 | 0.12 | 0.09 | 0.13 |
| | total impuri ties | 0.47 | 0.44 | 0.37 | 0.34 | 0.33 | 0.28 | 0.25 | 0.29 |
| 60°C, 10 d | pH | 8.4 | 8.3 | 8.2 | 8.2 | 8.2 | 8.3 | 8.2 | 8.4 |
| | impuri tyA | 0.45 | 0.58 | 0.29 | 0.51 | 0.44 | 0.26 | 0.53 | 0.55 |
| | total impuri ties | 0.60 | 0.69 | 0.45 | 0.66 | 0.59 | 0.42 | 0.70 | 0.71 |

### Example 15

Preparation process of formula for inhalation: a 0.4 mg/ml sodium hydroxide aqueous solution was prepared, sodium chloride was added as 9 mg/ml, sivelestat sodium tetrahydrate was added as 10 mg/ml, and the system was stirred to dissolve and clarify; the drug solution had a pH of 7.3, and was vialed as 5 mL and lyophilized.

### Example 16

Preparation process of formula for inhalation: the drug solution of Example 15 was added with polysorbate-80 as 0.2 mg/ml, and stirred to dissolve and clarify; the drug solution had a pH of 7.3, and was vialed as 5 mL and lyophilized.

### Example 17

Preparation process of formula for inhalation: a 1.1 mg/ml sodium hydroxide aqueous solution was prepared, sodium chloride was added as 9 mg/ml, polysorbate-80 was added as 0.2 mg/ml, sivelestat sodium tetrahydrate was added as 20 mg/ml, and the system was stirred to dissolve and clarify; the drug solution had a pH of 7.8, and was vialed as 5 mL and lyophilized.

The stability results of Examples 15-17 are shown in the table below.

| Formula | | Example 15 | Example 16 | Example 17 |
|---|---|---|---|---|
| sivelestat sodium | | 10 mg | 10 mg | 20 mg |
| sodium hydroxide | | 0.4 mg | 0.4 mg | 1.1 mg |
| sodium chloride | | 9 mg | 9 mg | 9 mg |
| polysorbate-80 | | - | 0.2 mg | 0.2 mg |
| water | | 1 ml | 1 ml | 1 ml |
| pH of drug solution | | 7.3 | 7.3 | 7.8 |

| Quality evaluation of lyophilized sample | | | | |
|---|---|---|---|---|
| Evaluated item | | Example 15 | Example 16 | Example 17 |
| Redissolving time | | 28 s | 20 s | 17s |
| Day 0 | osmotic pressure | 282 | 274 | 294 |
| | pH | 7.3 | 7.3 | 7.8 |
| | impurity A | 0.45 | 0.45 | 0.30 |
| | total impurities | 0.60 | 0.61 | 0.45 |
| 60°C, 10 d | pH | 7.3 | 7.3 | 7.8 |
| | impurity A | 0.82 | 0.86 | 0.67 |
| | total impurities | 1.01 | 1.15 | 0.88 |

By adjusting the proportions of raw materials and auxiliary materials in the formula, vialing the drug solution as 4 ml, and performing lyophilization, the formulas of Examples 18-20 were obtained; by vialing the drug solution as 2.5 ml, and performing lyophilization, the formulas of Examples 21-22 were obtained, as shown below.

| Formula | | Example 18 | Example 19 | Example 20 | Example 21 | Example 22 |
|---|---|---|---|---|---|---|
| sivelestat sodium | | 25 mg | 25 mg | 25 mg | 20 mg | 20 mg |
| sodium hydroxide | | 1.4 mg | 1.4 mg | 1.6 mg | 1.2 mg | 1.2 mg |
| sodium chloride | | 6.5 mg | 6.5 mg | 6.5 mg | 12 mg | 12 mg |
| polysorbate-80 | | - | 0.05 mg | 0.05 mg | - | 0.08 mg |
| pH of drug solution | | 7.8 | 7.8 | 8.2 | 7.8 | 7.8 |
| water | | 1 ml | 1 ml | 1 ml | 1 ml | 1 ml |

| Quality evaluation of lyophilized sample | | | | | | |
|---|---|---|---|---|---|---|
| Redissolving time | | 35 s | 19 s | 18 s | 34 s | 17 s |
| Da y0 | osmotic pressure | 300 | 302 | 305 | 297 | 296 |
| | pH | 7.8 | 7.8 | 8.2 | 7.8 | 7.8 |
| | impurity A | 0.43 | 0.44 | 0.51 | 0.45 | 0.46 |
| | total impuriti es | 0.60 | 0.61 | 0.66 | 0.62 | 0.63 |
| 60 °C, 10 d | pH | 7.8 | 7.8 | 8.2 | 7.8 | 7.8 |
| | impurity A | 0.44 | 0.48 | 0.66 | 0.50 | 0.52 |
| | total impuriti es | 0.61 | 0.65 | 0.81 | 0.67 | 0.69 |

### Example 23

### Tests for atomizing properties

Method for measuring the particle size of the present application: the lyophilized samples of Reference Example 1, Example 9, Example 10 and Example 11 were redissolved with water to prepare into drug solutions, and atomized by a PARI eflow atomizer, and the mass median aerodynamic diameter (MMAD) was measured by a new generation of pharmaceutical cascade impactor (NGI) with a flow rate of 15 L/min. The results show that the MMAD results are 8.047 µm, 3.921 µm, 2.005 µm and 2.056 µm, respectively, indicating that the pharmaceutical composition for inhalation of the examples of the present application can obtain the particle size which satisfies the medical requirements, and when the composition contains polysorbate-80, the particle size distribution will be more uniform after atomization.

The samples of Examples 18-22 were all redissolved with 4 ml of water, and tested for total delivery percentage and fine particle fraction. The total delivery percentage is the proportion of the delivery amount through the inhaling nozzle in the total drug amount when the drug solution is atomized in the atomizing cup; the fine particle fraction refers to the proportion of the delivery drug with a particle size of less than 5 µm.

| Atomizing properties | | | | | |
|---|---|---|---|---|---|
| | Example 18 | Example 19 | Example 20 | Example 21 | Example 22 |
| Total delivery percentage (%) | 37 | 44 | 43 | 36 | 42 |
| Fine particle fraction (%) | 65 | 80 | 78 | 66 | 79 |
| MMAD (µm) | 3.4 | 2.1 | 2.2 | 3.5 | 2.1 |

### Animal experiment

Based on the formula of Example 19, the lung exposure amounts of SD rats are compared between single injection administration of 5 mg/kg and inhalation administration of 5 mg/kg. Compared with the injection administration on the market, inhalation administration increases the exposure of drugs in lung by 20.5 times, and significantly prolongs the time for drugs to maintain the effective concentration in lung.

| Administration route | Concentration in lung µg/g | | | | AUC_{0.083-1h} (µg^{∗}h/g) |
|---|---|---|---|---|---|
| | 0.083 h | 0.167 h | 0.5 h | 1 h | |
| Injection (n=3) | 4.12 | 2.63 | 1.52 | 0.51 | 1.5 |
| Inhalation (n=3) | 35.4 | 47.7 | 25.1 | 8.5 | 30.7 |

The pharmaceutical composition for inhalation of the present application has good stability, and the particles atomized by the atomizing device can maintain uniformity. The pharmaceutical composition for inhalation can be used for inhalation administration treatment of patients with lung diseases, avoiding 24-hour continuous drip administration, and obviously improving the convenience of administration. The animal experiment shows that the inhalation composition of the present application significantly improves the drug concentration in lung and AUC value compared with the injection administration.

## Claims

1. A pharmaceutical composition for inhalation, comprising sivelestat sodium or a hydrate thereof, a pH-adjusting agent and an osmotic pressure-adjusting agent, wherein the pH-adjusting agent is capable to keep sivelestat sodium contained in the pharmaceutical composition having a solubility of more than or equal to 2.5 mg/mL, and the pharmaceutical composition for inhalation has an osmolality of 250-450 mOsm/kg.

2. The pharmaceutical composition for inhalation according to claim 1, wherein the pharmaceutical composition for inhalation further has at least one of the following features:
(1) the pharmaceutical composition for inhalation has a pH of 3.0-10.0;
(2) the pharmaceutical composition for inhalation has a particle size of 0.1-10.0 µm after being atomized by an atomizing device;
(3) a surfactant is optionally included.

3. The pharmaceutical composition for inhalation according to claim 1, wherein the pH-adjusting agent is capable to keep sivelestat sodium or the hydrate thereof contained in the pharmaceutical composition having a solubility of more than or equal to 5 mg/mL.

4. The pharmaceutical composition for inhalation according to claim 1, wherein the pH-adjusting agent is capable to keep an aqueous solution of the pharmaceutical composition for inhalation at pH 5.0-9.0, and/or pH 5.5-8.5.

5. The pharmaceutical composition for inhalation according to claim 1, wherein the pharmaceutical composition for inhalation has an osmolality of 250-400 mOsm/kg, preferably 250-350 mOsm/kg.

6. The pharmaceutical composition for inhalation according to claim 1, wherein the pharmaceutical composition for inhalation has a particle size of 0.5-7.0 µm after being atomized by an atomizing device.

7. The pharmaceutical composition for inhalation according to claim 1, wherein the composition is administered by inhalation after being dissolved in a carrier and atomized by an atomizing device.

8. The pharmaceutical composition for inhalation according to claim 1, wherein the carrier is water.

9. The pharmaceutical composition for inhalation according to claim 1, wherein the pH-adjusting agent has a content of 0.1-30.0 mg/mL, preferably 0.1-25.0 mg/mL.

10. The pharmaceutical composition for inhalation according to claim 1, wherein the surfactant has a content of 0.01-10.0 mg/mL, preferably 0.01-5.0 mg/mL.

11. A pharmaceutical composition for inhalation, comprising sivelestat sodium or a hydrate thereof, a pH-adjusting agent and an osmotic pressure-adjusting agent, wherein the pH-adjusting agent is capable to keep sivelestat sodium or the hydrate thereof contained in the pharmaceutical composition having a solubility of more than or equal to 5 mg/mL, and the pharmaceutical composition for inhalation has an osmolality of 250-400 mOsm/kg, and the pharmaceutical composition for inhalation has at least one of the following features:
(1) the pharmaceutical composition for inhalation has a pH of 5.0-9.0;
(2) the pharmaceutical composition for inhalation has a particle size of 0.5-7.0 µm after being atomized by an atomizing device;
(3) a surfactant is optionally included.

12. The pharmaceutical composition for inhalation according to claim 1 or 11, wherein the osmotic pressure-adjusting agent is optionally selected from sodium chloride and glucose.

13. The pharmaceutical composition for inhalation according to claim 1 or 11, wherein the surfactant is optionally selected from sodium lauryl sulfate and Tween 80.

14. A pharmaceutical composition for inhalation, comprising sivelestat sodium or a hydrate thereof, a pH-adjusting agent, an osmotic pressure-adjusting agent and a surfactant, wherein the pH-adjusting agent is capable to keep sivelestat sodium or the hydrate thereof contained in the pharmaceutical composition having a solubility of more than or equal to 5 mg/mL, and the pharmaceutical composition for inhalation has an osmolality of 250-350 mOsm/kg, and the pharmaceutical composition has a pH of 5.0-9.0.

15. A pharmaceutical composition for inhalation, comprising sivelestat sodium or a hydrate thereof, a pH-adjusting agent, an osmotic pressure-adjusting agent and a surfactant, wherein the pH-adjusting agent has a content of 0.1-25.0 mg/mL, the surfactant has a content of 0.01-5.0 mg/mL, an osmolality is 250-350 mOsm/kg, and a pH is 5.0-9.0.

16. The pharmaceutical composition for inhalation according to claim 14 or 15, wherein the osmotic pressure-adjusting agent is sodium chloride, and the surfactant is polysorbate-80.

17. Use of a pharmaceutical composition for inhalation in preparing a drug for treating a disease, wherein the pharmaceutical composition for inhalation is mixed with water and then atomized by an inhaler and inhaled, and the disease is a lung disease.
